(19) Europäisches Patentamt
European Patent Office
Office européen des brevets

(11) **EP 2 788 046 B1**

(12) **EUROPÄISCHE PATENTSCHRIFT**

(45) Veröffentlichungstag und Bekanntmachung des Hinweises auf die Patenterteilung:
**02.11.2016   Patentblatt 2016/44**

(21) Anmeldenummer: **13821827.6**

(22) Anmeldetag: **23.12.2013**

(51) Int Cl.:
*A61M 5/145* *(2006.01)*        *A61M 5/142* *(2006.01)*

(86) Internationale Anmeldenummer:
**PCT/EP2013/077950**

(87) Internationale Veröffentlichungsnummer:
**WO 2014/102259 (03.07.2014 Gazette 2014/27)**

(54) **PUMPE FÜR MEDIZINISCHE ZWECKE**

PUMP FOR MEDICAL PURPOSES

POMPE POUR BUTES MÉDICALES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.12.2012   DE 102012113087**

(43) Veröffentlichungstag der Anmeldung:
**15.10.2014   Patentblatt 2014/42**

(73) Patentinhaber: **B. Braun Melsungen AG**
**34212 Melsungen (DE)**

(72) Erfinder:
  • **HEITMEIER, Rolf**
    **34225 Baunatal (DE)**
  • **NIEDENZU, Dominik**
    **34131 Kassel (DE)**
  • **WOLFRAM, Berthold**
    **34212 Melsungen (DE)**
  • **SCHWALM, Matthias**
    **34613 Schwalmstadt (DE)**
  • **ROSENKRANZ, Heiko**
    **34593 Knüllwald (DE)**
  • **STEGER, Jürgen**
    **34327 Körle (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
WO-A1-01/21525        WO-A1-86/01115
WO-A1-2006/000415     WO-A1-2009/017487
WO-A2-2009/026060     DE-A1- 19 919 572
US-A- 5 921 951

**Beschreibung**

Die vorliegende Erfindung betrifft eine medizinische Pumpe zum Fördern von Fluiden (Flüssigkeiten) mit möglichst hoher Volumen- und Druckkontrolle bei gleichzeitiger Anwendung eines Durchfluss-Einmalartikel-Prinzips.

Hintergrund der Erfindung

Im klinischen Alltag werden speziell bei der intensivmedizinischen Behandlung von Patienten zur kontinuierlichen oder kurzintervallartigen Verabreichung von Wirkstoffen sogenannte Infusionspumpen eingesetzt. Hierbei handelt es sich üblicherweise um Spritzenpumpen oder um volumetrische Pumpen (z.B. Schlauchpumpen, peristaltische Quetschpumpen, Rollenpumpen, etc.). Diese zwei Pumpentypen unterscheiden sich grundlegend durch die größte wählbare Förderrate, also den maximal möglichen Volumenstrom, durch das maximal ohne Einmalartikelwechsel verabreichbare Gesamtvolumen, durch das Förderprofil und durch die Genauigkeit (z.B. Dosiergenauigkeit). Vereinfacht dargestellt, gelten für diese Pumpen die folgenden Auswahlkriterien:

- Spritzenpumpen finden Anwendung für:

Hohe Anforderungen an die Volumenstromgenauigkeit (z.B. hohe Dosierpräzision über die Zeit und/oder hohe Gleichförmigkeit der Förderung),
Hohe Anforderungen an das Druckprofil (z.B. keine Druckeinbrüche durch z.B. "Rückzugsphasen"),

Kleinere Volumenströme,

Hohe Langzeitkonstanz im Volumenstrom (z.B. keine Auswirkungen von Alterungs- und/oder Ermüdungsprozessen, z.B. durch das Walken eines Kunststoffschlauchs und/oder kein Einfluss durch das "Wegfließen" eines amorphen Kunststoffschlauchs) und geringes Applikationsvolumen per Einmalartikel (z.B. Spritze).

- Volumetrische Pumpen finden Anwendung für:

Geringere Anforderungen an die Volumenstromgenauigkeit (niedrigere Dosierpräzision über die Zeit),
Geringere Anforderungen an das Druckprofil (z.B. Druckschwankungen durch "Rückzugsphasen")

Große Volumenströme,

Geringere Langzeitkonstanz und

Gesteigertes Applikationsvolumen per Einmalartikel (z.B. Schlauch - z.B. durch Anwendung eines Durchflusspumpenprinzips, dann können beliebige Container(größen) vor der Pumpe verwendet und ohne Schlauchwechsel ausgetauscht werden).
Dies hat zur Folge, dass sich technologisch zwei unterschiedliche Pumpensysteme (gemäß unterschiedlicher Pumpverfahren) im Markt etabliert haben mit unterschiedlichen (bisher nicht kombinierbaren) Leistungsprofilen und daher auch unterschiedlichen Anwendungssituationen.
Im Konkreten hat die Spritzenpumpe konzeptionell eine Kolben-/Zylinderanordnung (bildet bzw. begrenzt auch gleichzeitig das bevorratete Gesamtvolumen) und verschiebt motorisch über z.B. ein Rotations-Translations-Umwandlungsgetriebe den (Spritzen-) Kolben in dem (Spritzen-) Zylinder, wohingegen die volumetrische Pumpe beispielsweise ein Schlauchsegment durch Krafteinwirkung (Pumpenrotor mit umfangsbeabstandeten Quetschelementen) in fortlaufende, fluidgetrennte Fluidkammern unterteilt bzw. partiell verschließt (was auch als Okklusion bezeichnet wird) und diesen Verschluss z.B. zyklisch, beispielsweise als Peristaltik, in Richtung Pumpenausgang (d.h. z.B. in Richtung Patient) bewegt.

Stand der Technik

Aus dem Stand der Technik sind eine Vielzahl von unterschiedlichen Pumpenkonstruktionen sowohl für die Spritzenpumpe als auch für die volumetrische Pumpe auf dem medizinischen Anwendungsgebiet bekannt.

Charakteristisch ist bei den volumetrischen Pumpenkonstruktionen, dass zu verabreichende Fluide aus separaten Fluidbehältern angesaugt und dann in Richtung Patient druckgefördert werden, wobei die erforderliche Saugwirkung i.A. durch die Rückstellfähigkeit (Eigenelastizität) des für gewöhnlich als Einmalartikel vorgesehenen Quetschschlauchs erzeugt wird. Durch diesen Umstand ist der Volumenstrom signifikant von den Strömungswiderständen im Pumpenzulauf (Saugseite der Pumpe), der Höhendifferenz zwischen Behälter und Pump-/Saug-Mechanik und der (i.A. veränderlichen) Eigendynamik des Schlauchmaterials abhängig. Die absolute Genauigkeit bei gleichen Aufbau- und Umgebungsbedingungen ist hingegen grundsätzlich durch die Genauigkeit des Schlauchmaterials als solches (Wanddicke, Innenquerschnitt, Materialzusammensetzung/Materialqualität, etc.) begrenzt. Da ferner das mechanische Verschieben des Verschlusses/der Okklusionsstelle für das verwendete Schlauchmaterial einen erheblichen mechanischen Stress darstellt, verändert sich der Volumenstrom in Folge von Verschleiß-/Ermüdungs-/Alterungs-/Kunststofffließeffekten im Schlauchmaterial kontinuierlich über die Zeit.

Die US 2012/0141306 offenbart eine Fluidpumpe mit einer ersten Fluidkammer und einer davon durch Membranen getrennte zweite Fluidkammer, die dadurch fluidisch von der ersten Fluidkammer getrennt ist. Die erste Fluidkammer enthält ein Arbeitsfluid, das mittels einer Kolben-Einrichtung zu und von der ersten Membran bewegt werden kann. Dadurch wird die erste Membran verformt und die Verformung wird auf die Membran, die die zweite Fluidkammer abschließt, übertragen. Es kommt je nach Kolbenbewegung zu einem Saug- oder Pumpbetrieb. Die Membranen sind dabei über ein Vakuum miteinander gekoppelt. Durch Steuerung des Kolbenantriebs wird das Pumpvolumen, das beispielsweise einem Patienten zugeführt wird, gesteuert.

Die US 6,250,502 B1 offenbart eine Präzisionsabgabepumpe zur Abgabe von Medien mit geringer oder hoher Viskosität (Flüssigkeiten, Pasten) für die Verwendung in Halbleitergehäusen und / oder Halbleiteranordnung. Die Präzisions-Abgabepumpe nutzt ein erstes Arbeitsmedium um das Abgabevolumen eines zweiten Mediums, also dem eigentlichen Arbeitsmedium, zu steuern. Zwischen den beiden Medien befindet sich eine Trennschicht, damit das abzugebende Medium getrennt vom Arbeitsmedium ist.

Die EP0191071B1 offenbart eine Pumpvorrichtung zur Förderung von Flüssigkeiten die hochempfindlich gegen mechanische Beanspruchungen sind, wie beispielsweise Blut. In dieser Pumpvorrichtung wird durch einen Druckerzeuger, z.B. eine Kolbenpumpe, einem Hohlraum eine Arbeitsflüssigkeit zu- oder abgeführt. Entsprechend bewegt sich der flexible Wandteil des Arbeitshohlraumes hin- und her. Der flexible Wandteil des Arbeitshohlraumes steht flächig in adhäsivem Kontakt mit dem flexiblen Wandteil des Förderraumes. Dadurch wird diese Hin- und Herbewegung direkt auf die in der Förderkammer befindliche Förderflüssigkeit übertragen, sodass Saug- bzw. Druckbewegungen entstehen. Über entsprechende Ventile, z.B. Schlauchklemmen an den Ab- und Zuflüssen in die Förderkammer erfolgen die Saug- bzw. Ausstoßbewegungen.

Die US 5,921,951 offenbart eine Pumpvorrichtung zum Pumpen mit einer konstanten Pumprate. Dabei werden mehrere Einzelpumpsysteme vorgesehen und angesteuert, um einen konstanten Fluidablauf herbeizuführen. Die Pumpen weisen dabei zwei miteinander verbundene Trennwände auf. Ein Arbeitsfluid wird aus einer Fluid-Säule in die erste Fluidkammer gedrängt, wodurch die flexiblen Wände verformt und das Volumen der zweiten Fluidkammer verändert wird. Dies erzeugt den Ansaug- und Ausstoßprozess der Pumpe.

Die WO 2006/000415 A1 offenbart ein Injektionssystem zur Injektion von Flüssigkeiten innerhalb eines starken Magnetfelds, eines magnetischen Wechselfelds und/oder eines elektrischen Hochfrequenzfelds zur Verwendung mit einem medizinisch-technischen System mit einem gegen elektromagnetische Felder mittels einer Abschirmung abgeschirmten Raum, einer Injektionsvorrichtung, durch die zu injizierende Flüssigkeit in einen Patienten abgebbar ist, einer Antriebseinrichtung der Injektionsvorrichtung, mit der mindestens ein Förderelement zur Injektion verlagerbar ist und einer außerhalb des Raumes angeordneten Steuer- und Kontrolleinheit. Die Antriebseinrichtung arbeitet hydraulisch und deren mindestens eine Hydraulikleitung wird zu einer Druckerzeugungseinheit geführt. Durch die Verwendung von Hydraulik-Komponenten werden Batterie, Elektromotoren, Stromkabel, Injektionskontrolleinheit sowie die beiden Sende- und Empfangsgeräte innerhalb des Raumes überflüssig. Durch die Verwendung des Injektionssystems entstehen keine ungewünschten elektromagnetischen Felder, da für das Injektionssystem kein Strom und kein leitfähiges Material innerhalb des Raumes verwendet werden.

Die DE 199 19 572 A1 offenbart ein Verfahren sowie eine Vorrichtung zur Bestimmung von Gas in medizinischen Flüssigkeiten, insbesondere Dialyseflüssigkeit, Blut und dergleichen mit zwei voneinander getrennten Kammern. Ein Gemisch aus Gas und Flüssigkeit wird von einer Kolbenmembranpumpe gefördert und bei abgesperrter Pumpkammer einer Volumen- und Druckänderung unterworfen, wobei Betriebsparameter der Pumpe, die Volumen- und Druckänderung repräsentieren, erfasst werden. Aus den erfassten Betriebsparametern der Pumpe wird durch eine Auswerteeinheit der Gasanteil bestimmt.

Es besteht daher der grundsätzliche Bedarf an einer Art medizinischer Universalpumpe, in welcher die Anwendungsvorteile der beiden vorstehend beschriebenen Pumpentypen in einem einzigen Pumpenprinzip vereinigt sind. D.h. die medizinische Universalpumpe sollte eine verbesserte Volumenstrom- und Druckprofilkontrolle entsprechend einer Spritzenpumpe durch prinzipbedingt größere Unabhängigkeit von Einflussgrößen einer an sich bekannten volumetrischen Pumpe erreichen wie z.B.

- Strömungswiderstände im Fluidiksystem, insbesondere im Zulauf, aber auch im Ablauf,
- Einmalartikelgeometrie und -materialschwankungen,
- Schwankungen der Eigendynamik des Einmalartikel,
- Verschleiß-/Ermüdungs-/Alterungs-/Kunststofffließeffekte des Einmalartikels
- Druck im Fluidsystem, insbesondere vor und nach dem Pumpsegment und außerdem Umgebungsdruck
- Gerät-, Umgebungs- und Fluidtemperatur.

Ferner sollte die Universalpumpe beliebig große Gesamtvolumina fördern können, ohne den das Pumpsegment umfassenden Einmalartikel auszutauschen - wie das bei volumetrischen Pumpen beispielsweise durch Austausch z.B. eines Infusionsbeutels ohne Wechsel des z.B. Infusionsschlauchs mit Pumpsegment möglich ist.

Kurzfassung der Erfindung

Der vorliegenden Erfindung liegt daher die Aufgabe zugrunde, eine medizinische Pumpe bereit zu stellen, die positive Eigenschaften einer an sich bekannten Spritzenpumpe mit positiven Eigenschaften einer an sich bekannten volumetrischen Pumpe (Quetsch-/Schlauchpumpe) vereinigt.

Diese Aufgabe wird durch eine medizinische Präzisions-/Universalpumpe mit den Merkmalen des Anspruchs 1 gelöst. Vorteilhafte Ausgestaltungen der Erfindung sind Gegenstand der Unteransprüche.

Das Grundprinzip der erfindungsgemäßen medizinischen (Saug-/Druck-) Pumpe besteht in der Anordnung zweier getrennter hydraulischer/pneumatischer Systeme bzw. Hydraulik-/Pneumatikkreise. Das erste hydraulische (oder pneumatische) System (Kreis) dient als Primär-Energiequelle zur Bereitstellung einer Saug-/Druckkraft und kommt nicht mit dem Patienten zu verabreichenden Fluiden in unmittelbaren Kontakt (dies ist besonders vorteilhaft, da die Pumpe andernfalls nach bzw. vor jedem Gebrauch gereinigt und/oder desinfiziert werden müsste). Dieses erste System, welches analog zum Spritzenpumpensystem konzipiert ist und eine Art innerer Simulationskreis bildet, zeichnet sich durch eine hohe Präzision bei der Erzeugung, Messung und/oder Kontrolle von Volumenströmen und Drücken aus - "stromaufwärts" (vor dem Pumpsegment), im Pumpsegment und/oder "stromabwärts" (nach dem Pumpsegment). Das zweite hydraulische (oder pneumatische ) System (Kreis) dient als Sekundär-Energiequelle zum Ansaugen und Förden von dem Patienten zu verabreichenden Fluiden und ist daher als Durchfluss-Einmalartikel (analog dem volumetrischen Pumpenprinzip) konzipiert bzw. weist einen Einmalartikel mit einem Zu- und Ablauf auf. Das zweite System (bzw. der Einmalartikel des zweiten Systems) wird über/durch das erste (wieder verwendbare) System betätigt/betrieben.

Erfindungsgemäß ist das erste System wieder verwendbar und vorzugsweise direkt mit dem zweiten System bzw. dessen Einmalartikel in der Form gekoppelt, dass eine Volumenänderung im ersten System zu einer entsprechenden (ggf. gleichen oder jedenfalls vorhersagbaren und/oder ermittelbaren (beispielsweise können z.B. im ersten oder zweiten System eingeschlossene beispielsweise Gasblasen anhand ihrer Kompressionskurve vermessen, berücksichtigt und/oder kompensiert werden) Volumenänderung im zweiten System (Einmalartikel) führt. Somit wird dem zweiten System (Einmalartikel) im Wesentlichen die Genauigkeit des ersten Systems aufgeprägt.

Bei dem ersten System kann es sich um eine druckdichte Fluidkammer (d.h. ein in der Fluidkammer anliegender Unter-/Überdruck bleibt nach dem Erreichen des Gleichgewichtzustands im Wesentlichen stabil / alternativ: der Druckverlauf ist vorhersagbar) mit mindestens einer Membran oder einer membranähnlichen beweglichen und/oder verformbaren Wandung handeln. Die Kopplung zum zweiten System erfolgt über die Membran. Liegt die Membran des ersten Systems im Wesentlichen formschlüssig auf der Membran (bzw. membranähnlichen Wandung) des zweiten Systems an, gibt diese Druckänderungen im ersten System an das zweite System z.B. in Form einer Verformung und damit z.B. Vergrößerung oder Verkleinerung der Fluidkammer des zweiten Systems weiter. Die Kopplung zwischen erster und zweiter Fluidkammer kann dabei besonders vorteilhaft analog einer Druckdose ausgestaltet sein, d.h. der Formschluss zwischen den Membranen kann beispielsweise durch die Evakuierung oder Befüllung des Membranzwischenraums und/oder eines die (gefügten) Membranen umgebenden Raums, dessen Wandungen beispielsweise auch sowohl aus zum ersten System gehörenden Wandungen wie auch aus zum zweiten System gehörenden Wandungen bestehen kann (und damit z.B. erst dann eine evakuierbare/befüllbare Druckdose bildet, wenn der Einmalartikel des zweiten Systems in den Mehrwegartikel des ersten Systems eingelegt ist), bewerkstelligt, unterstützt und/oder verbessert sein/werden. D.h., die (feste) Kopplung zwischen dem ersten und zweiten System wird durch eine (druckdichte) Fluiddruckkammer mit zwei durch zumindest eine bewegbare Wand getrennte Druckräume erreicht. Dieses Kopplungskonzept eröffnet z.B. auch die prinzipielle Möglichkeit, dass z.B. jeder beliebig geformte, elastische Einmalartikel (z.B. Schlauch mit Schlauchinnenvolumen als der zweite Druckraum) eingeformt werden kann, wobei das Volumen des ersten Druckraums (als Bestandteil des ersten Systems) z.B. nach dem Spritzenpumpenprinzip veränderbar ist, indem das Volumen des zweiten Druckraums über das erste System komprimiert und/oder ausgedehnt wird. D.h., die Verformung des zweiten Systems (Einmalartikels) kann eine Volumenänderung (Erhöhung/Verringerung) des zweiten Druckraums bewirken. Somit kann durch geeignetes Aktivieren des ersten Systems über das zweite System sowohl volumengenau Fluid aus einem Behälter angesaugt (Ausdehnen des zweiten Druckraums durch Volumenverringerung bzw -verschiebung des/im ersten Druckraum(s)) wie auch verdrängt werden (Komprimieren des zweiten Druckraums durch Volumenvergrößerung bzw. -verschiebung des/im ersten Druckraum(s)). Aufgrund dieser Eigenschaft kann das Verfahren erfindungsgemäß z.B. auch stromaufwärts angeschlossene Spritzen (als spritzenpumpentypische Fluidbehälter) wie auch z.B. Flaschen oder Beutel (als für volumetrische Pumpen typische Fluidbehälter) durch die über das erste System kontrollierte Saugeigenschaften (unabhängig von den eingangs genannten Faktoren) entleeren.

Konkreter ausgedrückt wird die gestellte Aufgabe mittels einer medizinischen Fluidpumpe gelöst mit einem ersten inneren Fluidsystem unter anderem bestehend aus einer motorisch getriebenen Saug-/Druck- bzw. Volumenverschiebe-Einheit und einem ersten Fluidraum, der durch die Saug-/Druckeinheit fluidbefüll- und entleerbar ist. Des Weiteren hat die erfindungsgemäße Pumpe ein zweites, äußeres Fluidsystem bestehend aus einem zweiten Fluid-Druckraum, der über eine bewegbare Trennwand druck- und/oder volumendynamisch mit dem ersten Fluid-Druckraum fluiddicht gekoppelt ist und der über eine Ventileinrichtung vorzugsweise des zweiten Fluidsystems in Abhängigkeit der aktuellen Arbeitsphase der Saug-/Druckeinheit des ersten Fluidsystems wechselweise mit einer Saug- und einer Druckleitung verbunden ist.

Durch die Betätigung der Saug-/Druck-Einheit kann somit das Füllvolumen des ersten Fluid-Druckraums variiert werden, was sich über die bewegliche Trennwand auf den zweiten Fluid-Druckraum entsprechend überträgt. D.h., auch der zweite Fluid-Druckraum verändert entsprechend der Ausgleich-Bewegung der Trennwand sein Volumen und saugt so Fluid aus einem Vorratsbehälter über die Saugleitung an und/oder stößt Fluid über die Druckleitung in Richtung Patient aus. Die Präzision bei der Einstellung der dabei auftretenden Volumenströme wird im Wesentlichen über die Saug-/Druck-Einheit unabhängig von den ggf. schwankenden Materialeigenschaften z.B. der Trennwände erreicht. Gleichzeitig ermöglicht die fluiddichte Zweiteilung beider Systeme, das äußere System oder Teile davon als Einwegartikel zu konzipieren. Besonders vorteilhaft kann so z.B. verhindert werden, dass Teile des zweiten Systems, z.B. dessen Trennwand mit Fluid des ("Hydraulik-") Systems in Kontakt kommen. Damit kann z.B. auf mit einem solchen Fluidkontakt notwendige zusätzliche Arbeitsgänge wie z.B. regelmäßige Fluidnachfüllung und/oder Reinigung im Wesentlichen verzichtet werden. Ferner kann das System vorteilhaft als im Sinne der Sterilität geschlossenes, z.B. Einmalartikelsystem, ausgestaltet sein. Hierdurch kann z.B. auf andernfalls notwendige zusätzliche Arbeitsgänge wie spezielle Desinfektion/Sterilisation verzichtet werden.

Vorzugsweise hat das erste Fluidsystem innerhalb des ersten Druckraums einen Drucksensor und vorzugsweise einen Volumen/Massenstromsensor zwischen dem ersten Druckraum und der Saug-/Druckeinheit zur Steuerung der Saug-/Druck-Einheit oder deren Antriebs und/oder eine derart präzisen Antriebeinheit, dass ein solcher Volumen- oder Massenstromsensor nicht benötigt wird. Es können mit einem Druck- bzw. Volumen/Massenstromsensor aber auch mittels regelungstechnischer Maßnahmen z.B. solche Saug-/Druck-Einheiten (Zahnradpumpen, Flügelzellenpumpen, etc.) eingesetzt werden, die konstruktiv nicht die geforderte Präzision bzw. Gleichförmigkeit erreichen. Hierzu kann auch beispielsweise vorteilhaft eine Zwischenphase z.B. mit beiden Ventilen (stromauf- wie stromabwärts) geschlossen eingesetzt werden (es kann somit bei geschlossenen Ventilen beispielsweise auf einen bestimmten Druck in den Systemen gefahren werden, derart dass das sich bei anschließender Öffnung der jeweiligen Ventile ergebende Förderprofil (stromauf- und/oder stromabwärts) für den Anwendungsfall besonders günstig ist.

Erfindungsgemäß ist vorgesehen, dass ein zumindest abschnittsweise flexibler Fluidaufnahmekörper in den zweiten Fluiddruckraum eingesetzt wird, um mit der beweglichen Trennwand des ersten Systems in Anlage zu sein, wobei der Fluidaufnahmekörper an die Ventileinrichtung fluidangeschlossen sein kann. In diesem Fall kann z.B. der den zweiten Fluiddruckraum ausbildende Behälter wieder verwendet werden, da er nicht mit Fluid im zweiten Fluidsystem kontaminiert wird, wobei lediglich der separat einsetzbare Fluidaufnahmekörper (beispielsweise ein Schlauch oder Beutel) entsorgt werden muss.

Ein anderer Aspekt der vorliegenden Erfindung betrifft eben jenen Fluid-(aufnahme)-behälter der erfindungsgemäßen medizinischen Fluidpumpe, der zumindest eine flexible Außenwand hat, welche den Fluidbehälter zumindest in montiertem Zustand fluiddicht verschließt und welche ggf. mit der beweglichen Trennwand in Anlage bringbar ist.

Gemäß einem bevorzugten Aspekt der Erfindung ist es vorgesehen, dass die medizinische Fluidfördervorrichtung derart gestaltet ist, dass sich mittels eines Drucksensors im ersten Fluidsystem als Simulationskreis auch, jedenfalls im Wesentlichen, der Druck im zweiten Fluidraum des zweiten Fluidsystems und im stromaufwärtig und/oder -abwärtig angeschlossenen System ermitteln lässt.

Gemäß einem weiteren bevorzugten Aspekt der Erfindung ist es vorgesehen, dass sich mittels des Drucksensors im ersten Fluidsystem auch, jedenfalls im Wesentlichen, mindestens ein Beitrag einer und/oder beider Trennwände zum Druck in einer und/oder beiden Fluidräumen ermitteln lässt.

Gemäß einem weiteren bevorzugten Aspekt der Erfindung ist es vorgesehen, dass sich zwischen einer Ansaug- und Verdrängungsphase und/oder zwischen einer Verdrängung- und Ansaugphase eine Zwischenphase bewerkstelligen lässt, in der sowohl das stromaufwärtige Ventil wie auch das stromabwärtige Ventil geschlossen ist. Gemäß einem weitern bevorzugten Aspekt der Erfindung ist es vorgesehen, dass sich, jedenfalls im Wesentlichen, das Volumen ermitteln lässt, das dem ersten Fluidraum in einem bestimmten Zeitraum hinzugefügt oder entzogen wurde.

Gemäß einem weiteren bevorzugten Aspekt der Erfindung ist es vorgesehen, dass das dem Fluidraum während eines Zeitraums hinzugefügte oder entzogene Fluidvolumen aus den in diesem Zeitraum an der Pumpeinheit und/oder einer mit der Pumpeinheit gekoppelten Sensorik anliegenden Signalen, jedenfalls im Wesentlichen, ableitbar ist.

Gemäß einem weiteren bevorzugten Aspekt der Erfindung ist es vorgesehen, dass während einer Zwischenphase, jedenfalls im Wesentlichen, ein gewünschter Druck im zweiten Fluidraum angesteuert werden kann.

Gemäß einem weiteren Aspekt der Erfindung ist es vorzugsweise vorgesehen, dass ein bestimmter, während einer Zwischenphase angesteuerter Druck im zweiten Fluidraum, sich vorteilhaft auf die anschließende Ansaug- und/oder Verdrängungsphase und/oder auf das Druckprofil stromauf- und/oder stromabwärts auswirkt.

Gemäß einem weiteren bevorzugten Aspekt der Erfindung ist es vorgesehen, dass sich ein besonders gleichförmiges Druckprofil stromauf- und/oder stromabwärts bewerkstelligen lässt.

Gemäß einem weiteren bevorzugten Aspekt der Erfindung ist es vorgesehen, dass sich in der Zwischenphase zwei oder mehr Tupel ("Fluidvolumen im ersten Fluidraum", "Druck am Drucksensor") oder Ähnliches (z.B. Kolbenposition und Druck) ermitteln lassen.

Gemäß einem weiteren bevorzugten Aspekt der Erfindung ist es vorgesehen, dass die in der z.B. Zwischenphase ermittelten Tupel genutzt werden können, um die Konsistenz und/oder Alterung einer oder beider Trennwände und/oder die Dichtigkeit der Systeme und/oder die Dichtigkeit einer und/oder beider geschlossenen Ventileinrichtungen und/oder die Compliance der Systeme und/oder das Kompressionsverhalten des Fluids in den Systemen und/oder der stromauf- und/oder stromabwärtige Druck und/oder der Zusammenhang zwischen Fluidvolumen in den Fluidräumen und dem Beitrag einer oder beider Trennwandauslenkungen bzw. -verformungen zum Drucksignal am Drucksensor und/oder Ähnliches zu bestimmen.

Gemäß einem weiteren bevorzugten Aspekt der Erfindung ist es vorgesehen, dass aus dem Kompressionsverhalten, jedenfalls im Wesentlichen, das Verhältnis zwischen gasförmiger und flüssiger Phase in einem der beiden und/oder in beiden Fluidräumen bestimmt werden kann.

Gemäß einem weiteren bevorzugten Aspekt der Erfindung ist es vorgesehen, dass sich in der zweiten Fluidkammer festsitzende Gasblasen, z.B. bei bestimmten Ventilstellungen, z.B. durch Druckstöße im ersten System in die stromab- und/oder stromaufwärtigen Systeme bewegen lassen.

Gemäß einem weiteren bevorzugten Aspekt der Erfindung ist es vorgesehen, dass sich das ergebende Druckprofil stromauf- und/oder stromabwärts vorteilhaft auf die Ausgasung und/oder Agglomeration von Gas im Fluid des weiten Systems bzw. in einem an dieses System angeschlossenen System auswirkt.

Gemäß einem weiteren bevorzugten Aspekt der Erfindung ist es vorgesehen, dass sich Fehler in den stromaufwärts und/oder -abwärts angeschlossenen Systemen, wie z.B. Leitungsverschlüsse und/oder Leckagen, anhand der Druckprofile in den Ansaug- bzw. Verdrängungsphasen detektieren lassen.

Gemäß einem weiteren bevorzugten Aspekt der Erfindung ist es vorgesehen, dass sich ungewünschte Veränderungen (z.B. im Druck) durch z.B. Höhenverlagerung von Teilen der stromauf- und/oder stromabwärtig angeschlossenen Systeme kompensieren lassen.

Figurenbeschreibung

Die Erfindung wird nachstehend anhand bevorzugter Ausführungsbeispiele unter Bezugnahme auf die begleitenden Figuren näher erläutert.

Fig. 1 zeigt anhand von drei Betriebspositionen (drei Abbildungen) zunächst das Funktionsprinzip einer medizinischen Pumpe gemäß der vorliegenden Erfindung,

Fig. 2 zeigt anhand von zwei Betriebspositionen (zwei Abbildungen) ein erstes bevorzugtes Ausführungsbeispiel einer medizinischen Pumpe nach dem Funktionsprinzip gemäß der Fig. 1 unter Verwendung eines Standardeinmalartikels z.B. eines (z.B. Infusions-) Schlauchs und

Fig. 3 zeigt anhand von zwei Betriebspositionen (drei Abbildungen) ein zweites bevorzugtes Ausführungsbeispiel einer medizinischen Pumpe nach dem Funktionsprinzip gemäß der Fig. 1 unter Verwendung eines beispielhaften, speziellen, besonders vorteilhaften Einmalartikels

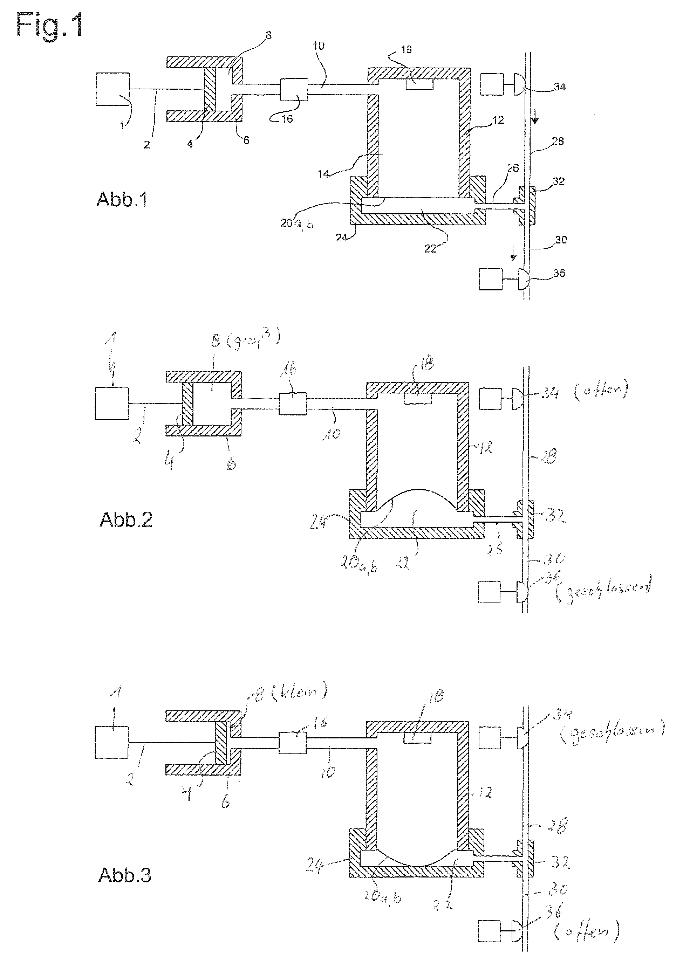

Gemäß der Fig. 1, Abb. 1 sieht das Grundprinzip der erfindungsgemäßen Pumpe (Universalpumpe) einen Antrieb 1 vor (beispielsweise einen Elektromotor oder dergleichen Leistungsquelle), der über eine Kraftübertragungseinheit 2 mit einem Translationskolben 4 wirkverbunden ist, welcher wiederum in einem Saug-/Druck-Zylinder 6 gelagert ist, um eine Fluidkammer 8 mit veränderbarem Volumen auszubilden. Diese Zylinder-Fluidkammer 8 ist über eine erste Fluidleitung 10 mit einem ersten Fluidbehälter 12 verbunden, in dem ein erster Druckraum 14 ausgebildet ist und in welchen die erste Fluidleitung 10 einmündet. Bei der Fluidkammer 8, der Fluidleitung 10 und dem ersten Druckraum 14 kann es sich auch beispielsweise um ein zusammenhängendes Volumen handeln, bei dem die Trennung in Fluidkammer, - leitung und Druckraum lediglich virtuell und gewissermaßen willkürlich wählbar ist und lediglich dem Zweck der besseren funktionalen Beschreibbarkeit dient.

In der ersten Fluidleitung 10 kann beispielsweise ein Volumenstromsensor 16 und in dem ersten Druckraum 14 kann beispielsweise ein Drucksensor 18 angeordnet sein. Die Kolben-Zylindereinheit 4, 6, die erste Fluidleitung 10 und der erste Druckraum 14 bzw. der erste Behälter 12 bilden zusammen mit den Sensoren 16, 18 und dem Antrieb 1 ein erstes hydraulisches System S1. Das erste hydraulische System S1 kann besonders vorteilhaft Teil eines Mehrwegartikels, beispielsweise einer Infusionspumpe, sein.

Der erste Druckraum 14 ist durch eine erste beweg-/ oder verformbare Wand 20a, z.B. eine Membran oder eine membranähnliche Vorrichtung abgeschlossen - die übrigen Wandungen des hydraulischen Systems S1 sind besonders vorteilhaft starr, so dass ein Druckanstieg oder -abfall in dem Druckraum nur zu einer Verformung der Wand 20a führt, sofern die Wand 20a hieran nicht, z.B. mechanisch von Außen, gehindert wird.

Ein zweites hydraulisches System S2 wird gebildet durch einen zweiten Druckraum 22 innerhalb eines zweiten Behälters 24, welcher z.B. an den ersten Fluidbehälter 12 anmontierbar/anmontiert bzw. einlegbar/eingelegt o.ä. ist. Das zweite hydraulische System S2 kann besonders vorteilhaft Teil eines Einmalartikels, beispielsweise eines Infusionssets, sein. Im Ausführungsbeispiel gehört beispielhaft ferner eine zweite Fluidleitung 26, ein Y-Stück 32, eine Saugleitung 28, eine Druckleitung 30 zum Einmalartikel und Ventile 34 und 36 gehören beispielhaft zum Mehrwegartikel, wie dies nachfolgend beschrieben wird.

Der zweite Druckraum 22 ist gemäß diesem Ausführungsbeispiel (aber nicht unbedingt erforderlich) durch eine zweite verformbare Wand 20b, z.B. eine Membran oder eine membranähnliche Vorrichtung abgeschlossen

Ferner ist der zweite Druckraum 22 beispielhaft über eine zweite Fluidleitung 26 an eine Saugleitung 28 und eine Druckleitung 30 angeschlossen oder anschließbar. Beispielsweise mündet die zweite Fluidleitung 26 in ein T- oder Y-Stück 32, das die Saugleitung 28 mit der Druckleitung 30 verbindet, wobei an und/oder in der Saug- und der Druckleitung 28, 30 jeweils ein Ventil (beispielsweise ein Rückschlagventil oder ein elektrisch angesteuertes 2/2-Wege/Schaltventil oder z.B. ein von einem Außen anlegbaren Aktuator z.B. abquetschbares Schlauchsegment oder Ähnliches) 34, 36 anordenbar/angeordnet ist. Besonders vorteilhaft handelt es sich um aktive Ventile, also solche, die z.B. derart gesteuert werden, dass z.B. eine gewünschte Strömung in die gewünschte eine Richtung, also beispielsweise von einem nicht weiter gezeigten Fluidvorratsbehälter/Container/Tank, z.B. einem Infusionsbeutel (Pumpensaugseite) in Richtung hin zu einem nicht weiter gezeigten Zielort, z.B. einem Patienten (Pumpendruckseite) entsteht. Alternativ hierzu sind aber auch andere Ventilkonstruktionen denkbar, wie beispielsweise ein Umschaltventil zur wahl-/wechselweisen Verbindung der zweiten Fluidleitung 26 mit der Saug- und Druckleitung 28,30.

Die erste bewegbare/verformbare Trennwand 20a ist besonders vorteilhaft mit der zweiten Trennwand 20b mechanisch reversibel koppelbar, z.B. derart, dass beide z.B. Membranen im Wesentlichen spaltfrei fügbar sind und die Trennwand 20b (beispielsweise aufgrund der in den Druckräumen 14 und 22 gegenüber einem während/durch die Verformung entstehenden Spalt zwischen den Membranen größeren Drücken) den Bewegungen/Verformungen der Trennwand 20a folgt.

Das Funktionsprinzip der erfindungsgemäßen Pumpe mit vorstehend beschriebenem konzeptionellem Aufbau lässt sich wie Folgt umschreiben:

Im Ruhezustand gemäß der Fig. 1, Abb. 1 ist der Volumenstrom im zweiten System S2 durch die geschlossene Stellung des stromabwärtigen Ventils 36 in Richtung hin zum z.B. Patienten (Druckseite) unterbrochen. Der Druckraum (Fluidraum) 22 ist zu Beginn entweder mit einer Flüssigkeit und/oder mit einem Gas gefüllt und kann initial entgast werden. In der weiteren Beschreibung wird aus Vereinfachungsgründen von einem im Wesentlichen entgasten, flüssigkeitsgefüllten zweiten System S2 ausgegangen. In diesem Zustand befindet sich die zweite bewegbare Wand, beispielsweise eine Membran, z.B. in ihrer (z.B. unbelasteten) Konstruktionslage. Das erste System S1 ist ebenfalls mit einer Flüssigkeit und/oder einem Gas gefüllt. Bei dem Fluid in S1 handelt es sich besonders vorteilhaft um ein im Wesentlichen inkompressibles Medium oder um ein Medium mit einer definierten und bekannten Kompressibilitätskurve. Der Kolben 4 kann sich zu diesem Zeitpunkt beispielsweise in einer vorgeschobenen Stellung mit kleinem Volumen innerhalb der Zylinderkammer 8 befinden. Das erste System S1 kann im Ruhezustand z.B. im Wesentlichen auf Atmosphärendruck sein.

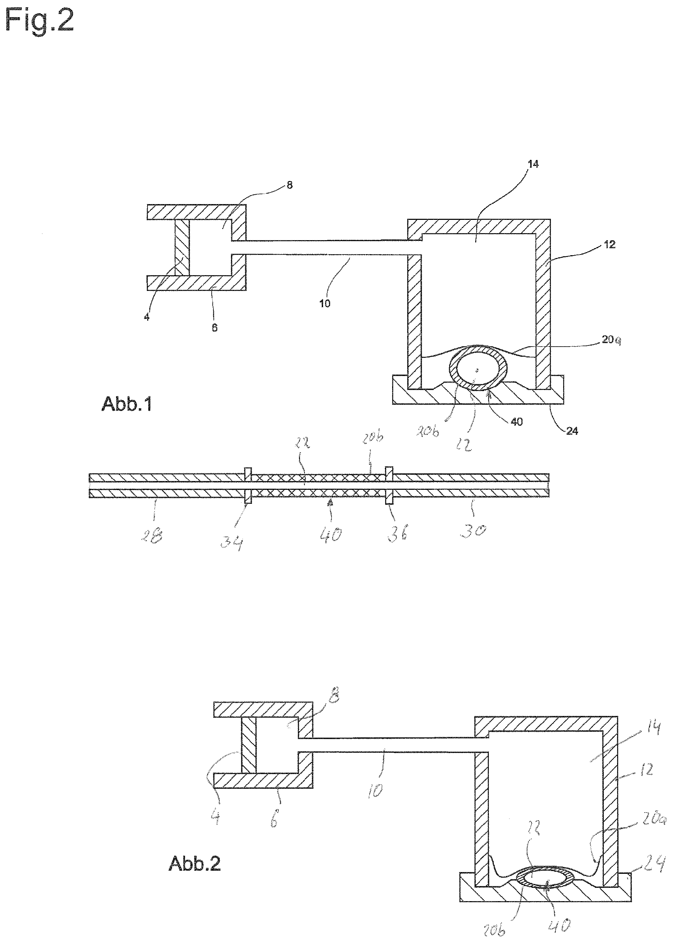

Die Fig. 1, Abb. 2 zeigt einen Ansaugzustand der erfindungsgemäßen Pumpe. In diesem ist/wird der Kolben 4 durch den Antrieb 1 zurückgezogen und damit das Volumen der Zylinderkammer 8 vergrößert. In Folge dieser Rückziehbewegung des Kolbens 4 strömt Fluid aus dem ersten Fluidraum 14 durch die erste Fluidleitung 10 in die Zylinderkammer 8, wobei der Volumenstrom durch einen Sensor 16 detektiert werden kann.

Der Abzug an Flüssigkeit aus dem ersten Druckraum 14 wird durch eine entsprechende Bewegung der Trennwand 20a zur Volumenverringerung des ersten Druckraums 14 kompensiert. Durch die Kopplung der Trennwände 20a mit 20b, folgt die Trennwand 20b der Bewegung der Trennwand 20a, wodurch gleichzeitig das Volumen des zweiten Druckraums 22 entsprechend (z.B. besonders vorteilhaft um das gleiche Volumenmaß) vergrößert wird. Demzufolge wird entsprechend der Volumenvergrößerungsbewegung der Trennwand 20b ein Fluid aus einem nicht gezeigten Vorratstank über die Saugleitung 28 und das geöffnete Saugventil 34 in den zweiten Druckraum 22 eingesaugt. Das Ventil 36 ist in dieser (Saug-)Phase weiterhin geschlossen, wie dies in der Fig. 1, Abb. 2 dargestellt ist.

Die Bewegung des Kolbens 4 kann über die Querschnittsfläche des Kolbens direkt proportional zum Volumenstrom durch die erste Fluidleitung 10 ausgestaltet sein. Dieser Volumenstrom kann über den Sensor 16 erfasst und/oder über beispielsweise eine Umdrehungs- und/oder Wegmessung am Kolben 4, an der Kraftübertragungseinheit 2 und/oder am Antrieb 1 ermittelt werden. Es ist also beispielsweise auch vorteilhaft denkbar, dass die Drehzahl des Antriebs oder die Schrittzahl eines Schrittmotors einen präzisen Bezug zum Volumenstrom herstellt.

Da die Kolbenrückbewegung einem Ansaugen von Flüssigkeit entspricht, wird durch den im ersten System S1 entstehenden verringerten Druck die bewegbare Wand 22 des zweiten Systems S2 nach innen in Richtung erstem Druckraum 14 verformt. Der Sensor 18 kann dabei den erzeugten verringerten Druck detektieren und hiermit beispielsweise vorteilhaft auch die Systemdichtigkeit der Systeme S1 und S2, der Ventile 34 und 36, sowie die Kompressibilitäten bzw. Compliances der vorgenannten bzw. deren Fluiden und auch z.B. die (Korrektheit der) Fügung der Trennwände 20a und 20b gegen eine Erwartungshaltung prüfen. Analoges gilt für die Kolbenvorwärtsbewegung. Besonders vorteilhaft können zudem hier nicht skizzierte Zwischenzustände, wie beispielsweise das gezielte vor-/rückwärts-Bewegen des Kolbens bei geschlossenen Ventilen 34 und 36 zur Erzeugung eines Druckanstiegs bzw. Druckabfalls zur Ermittlung der Dichtigkeiten, Kompressibilitäten und/oder Compliances der, insbesondere der vorgenannten, Bestandteile der erfindungsgemäßen (vorteilhaft Mehrweg-) Pumpe beziehungsweise des zugehörigen erfindungsgemäßen (vorteilhaft Einmal-) Artikels, genutzt werden. Hierdurch können beispielsweise vorteilhaft auch Luftblasen in den jeweiligen (ansonsten beispielsweise im Wesentlichen inkompressiblen) Fluiden detektiert und/oder vermessen werden und Ähnliches. Es können ferner z.B. vorteilhaft Leckagen, z.B. durch Risse in den Trennwänden, detektiert und/oder vermessen werden. Ebenso können aber auch z.B. Leitungsverschlüsse stromabwärts wie auch stromaufwärts bei den entsprechenden Ventilstellungen vorteilhaft detektiert werden. Durch die druckdichte/fluiddichte Kopplung des Druckraums 14 des ersten Systems S1 mit dem Druckraum 22 des zweiten Systems S2 strömt genau so viel Volumen an Fluid in den Druckraum 22 wie vom Kolben 4 in die Zylinderkammer 8 bewegt (angesaugt) wurde.

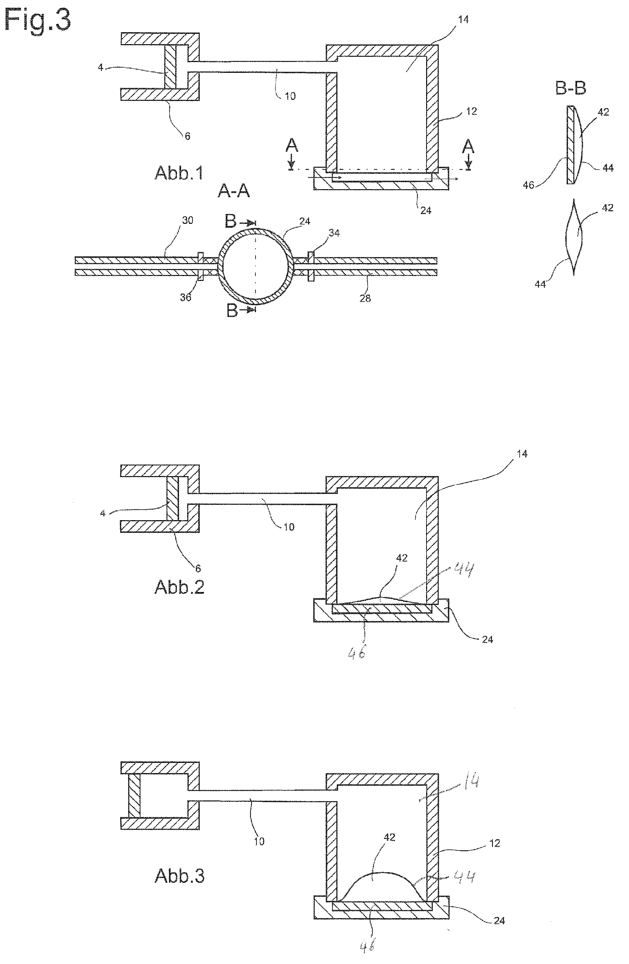

Gemäß der Fig. 1, Abb. 3 bewegt sich der Kolben 4 in die zur Abb. 2 entgegengesetzte Richtung zur Verkleinerung des Volumens der Zylinderkammer 8 und drückt damit eine entsprechende Menge an Fluid aus der Zylinderkammer 8 in den ersten Druckraum 14. Dies führt zu einer Bewegung bzw. Verformung der Trennwände 20a und 20b in Richtung zweiter Druckraum (Fluidraum) 22 und einer daraus resultierenden Fluidverdrängung aus dem Druckraum 22.

In dieser (Druck-) Phase ist das Saugventil 34 bereits geschlossen und statt dessen das Druckventil 36 geöffnet, sodass es zu einem definierten Volumenstrom an Fluid aus dem zweiten Druckraum 22 durch die Druckleitung 30 in Richtung hin zum Patienten kommt. Dieser Volumenstrom entspricht dem verschobenen (ausgedrückten) Volumen in der Kolben-/Zylindereinheit 4, 6.

Je nach Beweglichkeit (Elastizität) der Trennwände (z.B. Membranen) bauen sich im ersten Druckraum 14 Fluiddrücke auf, die von den im zweiten System S2 bestehenden Drücken überlagert werden. Befindet sich die Pumpe z.B. im Ruhezustand, gemäß der Fig. 1, Abb. 1, kann durch z.B. den Drucksensor 18 der Druck im ersten System S1 detektiert werden. Druckänderungen oder -pulsationen im ersten System S1 werden somit unmittelbar in das zweite System S2 übertragen und umgekehrt. Bei geeigneter Gestaltung der beweglichen Trennwand (Membran), also beispielsweise hochflexibel mit geringen Rückstellkräften und z.B. mit geringer Kompressibilität, sind damit, je nach Ventilstellung der Ventile 34 und 36, auch Änderungen in den an die Saugleitung 28 bzw. die Druckleitung 30 angeschlossenen Systemen ermittelbar. Hierbei könnte es sich stromaufwärts beispielsweise im Falle einer angeschlossenen (und nicht belüfteten) Infusionsglasflasche um einen stetig größer werdenden Unterdruck und stromabwärts beispielsweise im Falle eines angeschlossenen verstopften Infusionsfilters um einen stetig größer werdenden Überdruck handeln.

Durch die druckdynamische Kopplung der beiden Systeme S1 und S2 gemäß vorstehender Beschreibung (über die beweglichen Trennwände) sind durch den Sensor 18 auch z.B. während der Pumpphase Druckveränderungen sowohl im Pumpenzulauf (Saugseite) wie auch im Pumpenablauf (Patientenseite) detektier-/ermittelbar. Es können so z.B. auch Strömungswiderstände stromab wie auch stromauf (verursacht durch z.B. Verschlüsse jeglicher Art, Filter, verschlossene Rollenklemmen, abgeknickte Schläuche, etc.) detektiert werden.

Besonders vorteilhaft können die Druckmessungen bzw. -ermittlungen des jeweiligen Drucks im ersten Druckraum 14 (bzw. im ersten hydraulischen System S1), im zweiten Druckraum 22 (bzw. im zweiten hydraulischen System S2), im stromaufwärts an die Saugleitung 28 hydraulisch angeschlossenen System und im stromabwärts an die Druckleitung 30 hydraulisch angeschlossenen System verbessert werden, wenn der das zugehörige Drucksignal z.B. am Drucksensor 18 verfälschende Beitrag der Trennwände/Membranen 20a und 20b, der z.B. durch deren Vorspannungen und/oder beispielsweise durch die der jeweiligen Membranbewegung/-verformung entgegenwirkende Rückstellkraft begründet sein kann, ermittelt und berücksichtigt wird. Die Ermittlung diesen Beitrags z.B. an einer bestimmten ersten Position z1 des Translationskolbens 4 kann beispielsweise wie folgt vonstatten gehen (idealisiert und vereinfacht und lediglich mit beispielhaft anschaulichem Charakter dargestellt):

1.) Messung des Drucksignals am z.B. Drucksensor 18 bei geöffnetem Ventil 34 (stromaufwärts) und geschlossenem Ventil 36 (stromabwärts), der Messwert sei in der Folge mit Pu bezeichnet

2.) Messung des Drucksignals am z.B. Drucksensor 18 bei geschlossenem Ventil 34 (stromaufwärts) und geschlossenem Ventil 36 (stromabwärts), der Messwert sei in der Folge mit Pc bezeichnet

3.) Messung des Drucksignals am z.B. Drucksensor 18 bei geschlossenem Ventil 34 (stromaufwärts) und geöffnetem Ventil 36 (stromabwärts), der Messwert sei in der Folge mit Pd bezeichnet

Vereinfacht gilt dann:

$$1.) \; Pu(z1) = PUmgebung + Pstromaufwärts + PMembranbeitrag(z1)$$

$$2.) \; Pc(z1) = PUmgebung + \qquad\qquad PMembranbeitrag(z1)$$

$$3.) \; Pd(z1) = PUmgebung + Pstromabwärts + PMembranbeitrag(z1)$$

Somit sind Pstromaufwärts = Pu(z1) - Pc(z1) und Pstromabwärts = Pd(z1) - Pc(z1) ermittelbar. Zieht man ferner eine weitere analoge Messung an einer zweiten Kolbenposition z2 heran, lässt sich z.B. mit Pu(z1)-Pu(z2) = PMembranbeitrag(z1)- PMembranbeitrag(z2) beispielsweise auch der Unterschied in den Beiträgen der Membranen zum Drucksignal an den beiden Kolbenpositionen z1 und z2 ermitteln.

Es ist nicht wichtig, ob die hier angenommenen Gleichungen ein reales System korrekt beschreiben (das hängt dann konkret von weiteren Faktoren, wie deren Compliances, der Ventilschaltreihenfolge und -Zeitverhalten und dem übrigen Systemzeitverhalten usw. ab) - es ist lediglich wichtig, dass mittels Messung des Drucksignals z.B. am Drucksensor 18 bei verschiedenen Ventilkonstellationen und gegebenenfalls bei verschiedenen Positionen des Kolbens 4 (bzw. der Membranen) so viele Gleichungen aufgestellt werden können, dass mehr Gleichungen als Unbekannte vorliegen - die Gleichungen demnach lösbar sind und die in den Gleichungen vorkommenden Unbekannten (z.B. Umgebungsdruck, Druck im System stromaufwärts, Membranrückstellkraft an einer bestimmten Position * Fläche, Druck im System stromabwärts etc.) ermittelbar sind.

Selbstverständlich kann ferner die vierte Ventilkonstellation: beide Ventile geöffnet ebenfalls herangezogen werden.

Solche oder ähnliche Messungen können beispielsweise auch genutzt werden, die Mehrwegartikelmembran 20a z.B. während der Produktion und/oder während eines Geräteservice (Inspektion, Selbstkalibrierung, Selbsttest, etc.) "zu kalibrieren" - sprich deren Verhalten zu vermessen und beispielsweise im Mehrwegartikel (z.B. in der Infusionspumpe) abzuspeichern (z.B. dauerhaft).

Solche oder ähnliche Messungen können beispielsweise ferner genutzt werden, um z.B. vor Beginn der Förderung (z.B. vor dem Infusionsstart z.B. nach Einlegen des Einmalartikels in die Infusionspumpe) die Einwegartikelmembran 20b z.B. initial "zu kalibrieren" - sprich deren Verhalten zu vermessen und beispielsweise im Mehrwegartikel (z.B. in der Infusionspumpe) zu hinterlegen (z.B. lediglich temporär für die Zeitdauer des Einsatzes dieses Einmalartikels).

Solche oder ähnliche Messungen können beispielsweise aber auch ebenso während des Betriebs erfolgen - hierzu können vorteilhaft z.B. zwischen den in Figur 1 gezeigten Phasen Ansaugen (Abb. 2 - entspricht Pu) und Verdrängen (Abb. 3 - entspricht Pd) hier nicht skizzierte Zwischenphasen eingefügt werden, die z.B. Pc entsprechen können. Ferner kann in diesen Zwischenphasen der Kolben auch beispielsweise vor und/oder zurückbewegt werden, um Messungen bei verschiedenen Kolbenpositionen zur Ermittlung einer Größe heranziehen zu können.

Außerdem können besonders vorteilhaft auch z.B. Druckdifferenzen zwischen S1, S2 und den S2 angeschlossenen Systemen stromaufwärts und stromabwärts durch das entsprechende Fahren des Kolbens 4 z.B. während solchen Zwischenphasen z.B. bei geschlossenen Ventilen 34 und 36 ausgeglichen werden - diese und ähnliche Prozedere ermöglichen die Bewerkstelligung eines sehr gleichförmigen Druckprofils in allen genannten hydraulischen Zweigen, insbesondere aber stromauf- und abwärts. Somit ist auch ein sehr gleichförmiges Förderprofil machbar. Ferner erhöht ein solches Vorgehen beispielsweise auch die leistbare Präzision signifikant. Gleichförmige Druckprofile vermindern zudem die Ausgasung in Fluiden und/oder die Agglomeration von Mikroblasen zu Makroblasen.

Ganz besonders vorteilhaft lassen sich also praktisch alle für den Pumpvorgang interessanten Größen mittels einem einzigen Drucksensor 18 und zweier Ventile 34 und 36 überprüfen und steuern.

Sehr vorteilhaft ist auch die Möglichkeit, eine mögliche Veränderung z.B. der Membranverhaltensweisen (z.B. durch Materialermüdung und/oder Defekt) zu detektieren und/oder zu kompensieren - vor Start, während der Förderung und/oder nach Beendigung der Förderung. Es sind demnach mit solchen Vorgehensweisen auch umfassende Selbsttests möglich.

Beispiele für detektierbare Systemänderungen sind: Höhenverlagerung der stromaufwärtigen Fluidquelle, der stromabwärtigen Fluidsenke und/oder des Mehrwegartikels o.ä., Veränderung der Strömungswiderstände z.B. stromauf- und/oder abwärts (und damit z.B. Leitungsverschlüsse und/oder -leckagen), Gegendrücke z.B. stromabwärts (z.B. auch Patientenblutdruck o.ä.), Undichtigkeiten im ersten und/oder zweiten hydraulischen System, Materialermüdungen und/oder -defekte wie z.B. Risse und/oder Löcher o.ä. in den Trennwänden 20a und/oder 20b, aber auch z.B. Veränderungen des Fluids (z.B. aufgrund einer neuen

Infusionsflüssigkeitszusammensetzung und/oder aufgrund von z.B. Luftblasen z.B. in S1 und/oder in S2). Andererseits lässt sich auch die Plausibilität des Drucksensorsignals und damit der Funktionsfähigkeit des Drucksensors überprüfen.

Nach der Pump- bzw. Verdrängungsphase gemäß der Fig. 1, Abb. 3 geht die Pumpe wieder in die Ansaugphase gemäß der Fig. 1, Abb. 2 über, wobei sich der Phasenwechsel zwischen Ansaugen und Verdrängen vorzugsweise kontinuierlich oder intervallartig wiederholt. Insbesondere bei der kontinuierlichen Wiederholung kann ein diskontinuierlicher Volumenstrom dadurch reduziert oder weitgehend vermieden werden, indem die prinzipbedingten Totphasen, die insbesondere stromab (auf der Pumpendruckseite) störend sein können, durch unterschiedliche Bewegungsgeschwindigkeiten des Kolbens 4 (hohe Ansauggeschwindigkeit/niedrige Verdrängungsgeschwindigkeit) minimiert werden. Ferner kann das Prinzip auch derart erweitert werden, dass während einer Totphase ("Rückzugsphase", "Ladephase", "Ansaugphase") ein zweite Pumpe gemäß dieser Erfindung fördert und umgekehrt (sozusagen eine Doppel- oder Zweikolbenpumpe).

Wie außerdem anhand der Fig. 1 zu entnehmen ist, erscheint es vorteilhaft, zumindest den zweiten Fluidbehälter 24 einschließlich der Membran (bewegliche Trennwand) 20b als ein separates Bauteil, insbesondere als Einwegartikel auszubilden, wobei vorzugsweise das gesamte zweite System S2 als Einwegartikel vorgesehen ist. D.h., dass in diesem Fall der zweite Behälter 24 vom ersten Behälter 12 (z.B. reversibel) trennbar gekoppelt sein kann, derart, dass eine Druckänderung in dem ersten Druckraum 14 zu einer Ausgleichsbewegung der Trennwand 20a und b und damit einer Volumenänderung des zweiten Druckraums 22 führt.

Beispielsweise können beide Behälter ineinander gesteckt und/oder geschraubt und/oder zusammengepresst sein. Die Behälter können aber auch aneinander geflanscht werden. Durch die vorgesehenen zwei z.B. parallelen Trennwände/Membranen, die jeweils den ersten, bzw. den zweiten Druckraum abdichten, müssen bei einer Demontage beider Behälter die Druckräume z.B. nicht entleert werden.

Im Nachfolgenden werden technische Hinweise auf konkrete technische Umsetzungen des vorstehenden Pumpenprinzips anhand der Fig. 2 und 3 gegeben.

Insbesondere, wenn unter sterilen Bedingungen gefördert werden soll, kann es auch vorteilhaft sein, wenn sich das Konzept eines Einmalartikels nicht auf das gesamte zweite System S2 einschließlich des Behälters 24 bezieht, sondern z.B. auf speziell an den zweiten Behälter angepasste, diesen sozusagen auskleidende Elemente reduziert.

Gemäß der Fig. 2, Abb. 1 und 2 wird der eigentliche zweite Fluid-Druckraum quasi durch z.B. einen (z.B. Infusions-) Schlauch 40 als Fluidaufnahmekörper gebildet, der in den zweiten Behälter 24 einlegbar ist und formschlüssig an die bewegbare Trennwand/Membran 20a angekoppelt ist. D.h., die Wandung des Schlauchs 40 entspricht somit der zweiten Trennwand/Membran 20b, wobei für den Fall, dass das Schlauchmaterial hinreichend flexibel/verformbar ist, die Trennwand 20a den Schlauch 40 entsprechend deformieren kann, sodass ein z.B. direkt proportionaler Volumenstrom erzeugbar ist. Damit muss nur der Schlauch 40 beispielsweise samt daran angeschlossener Saug-/Druckleitungen 28, 30 und ggf. auch Ventile 34, 36 als Einwegartikel konzipiert sein.

Alternativ ist es gemäß der Fig. 3, Abb. 1 bis 3 auch beispielsweise vorgesehen, den zweiten Behälter 24 z.B. mit einem kissenartigen Inlay 42 (z.B. lose) auszulegen. Das z.B. beutelförmige Kissen 42 kann in Konstruktionslage (z.B. drucklos) ein zu dem zweiten Behälter gleiches Volumen (füllt diesen daher im Wesentlichen vollständig aus) haben und z.B. aus einer flexiblen/elastischen Folie/Membran 44, die randseitig an einer z.B. Platte 46 fluiddicht fixiert ist, bestehen (der Einmalartikel kann aber auch vorteilhaft bereits die Funktion des zweiten Behälters 24 abdecken - z.B. indem dessen Gehäuse fest und/oder reversibel und/oder dicht an den ersten Behälter 12 montierbar ist - mit z.B. dem Vorteil, dass kein zusätzliches Bauteil für den zweiten Behälter 24 notwendig ist). In den dadurch gebildeten Beutel- Innenraum (zweiter Druckraum) führt zumindest eine Öffnung (nicht weiter gezeigt) für die zweite Fluidleitung 26. Diese Öffnung kann beispielsweise in der Platte 46 ausgenommen sein. Es können aber auch beispielsweise zwei oder mehr Zugänge, z.B. einen jeweils für Zuleitung 28 und Ableitung 30 vorgesehen sein. Dabei sei darauf hingewiesen, dass anstelle der Platte 46 der Beutel auch ausschließlich aus dem flexiblen Material gefertigt sein kann. Bei dieser Ausführungsform wird somit die Bewegung der beweglichen Trennwand 20a unmittelbar auf den Beutel 42 für dessen entsprechende Volumenänderung übertragen.

Die vorstehend beschriebenen Ausführungsbeispiele sind so gewählt worden, dass die Funktionsweise der erfindungsgemäßen Pumpe in einfacher Weise darstellbar ist. Sie entsprechen daher nicht notwendiger Weise einer tatsächlichen Realisierungsform. So kann z.B. die getrennt dargestellte Kolben-/Zylindereinheit auch bereits Bestandteil des ersten Behälters sein. Ebenso ist es denkbar, dass der Kolben der Kolben-Zylindereinheit zwei oder mehrere erste Systeme mit Flüssigkeit füllt oder leert, an die wiederum jeweils ein zweites System anschließbar/angeschlossen ist.

Dies ist beispielsweise dann sinnvoll, wenn durch mehrere parallel geschaltete Systeme (bei entsprechender Phasenverschiebung) die Totphasen im vorstehend beschriebenen Ein-System-Ausführungsbeispiel minimiert werden sollen.

Auch sei darauf hingewiesen, dass die Kolben-/Zylindereinheit nur eine beispielhafte Lösung darstellt. Es ist genauso denkbar, dass jedes denkbare Pumpverfahren Anwendung findet wie beispielsweise eine Taumelscheibenpumpe, eine Flügelzellenpumpe, eine Zahnradpumpe, etc. Solche Pumpeinheiten, die per se keine ausreichende Volumenstromgenauigkeit erlauben (Membranpumpen, etc.) können erfindungsgemäß z.B. mit einem Volumenstromsensor und/oder Massenstromsensor oder Ähnlichem ausgestattet sein, um die gewünschte Genauigkeit regelungstechnisch herbeizuführen.

Bezugszeichenliste

| | |
|---|---|
| 1 | Antrieb |
| 2 | Kraftübertragungseinheit |
| 4 | Translationskolben |
| 6 | Zylinder |
| 8 | Fluidkammer |
| 10 | Fluidleitung |
| 12 | erster Fluidbehälter |
| 14 | erster Fluid-/Druckraum |
| 16 | Volumenstromsensor |
| 18 | Drucksensor |
| 20a,b | Trennwand |
| 22 | zweiter Fluid-/Druckraum |
| 24 | zweiter Behälter |
| 26 | zweite Fluidleitung |
| 28 | Saugleitung |
| 30 | Druckleitung |
| 32 | Y-Stück |
| 34,36 | Rückschlagventile |
| 40 | Fluidaufnahmekörper/Schlauch |
| 42 | Inlay/Kissen |
| 44 | Folie/Membran |
| 46 | Platte |

**Patentansprüche**

1. Medizinische Fluidfördervorrichtung mit

   - einem ersten Fluidsystem (S1) bestehend aus einer motorisch getriebenen Pumpeinheit (4, 6) und einem ersten Fluidraum (14), dem durch die Pumpeinheit (4, 6) Fluid hinzufüg- und entziehbar ist, und einer ersten beweg- und/oder verformbaren Trennwand (20a), und
   - einem zweiten, an das erste Fluidsystem (S1) ankoppelbaren Fluidsystem (S2) bestehend aus einem zweiten Fluidraum (22) und einer zweiten beweg- und/oder verformbaren Trennwand (20b), die mit der ersten Trennwand (20a) derart mechanisch oder fluidisch koppelbar ist, dass sich eine Volumen- und/oder Druckänderung im ersten Fluidraum (14) im Wesentlichen vorhersagbar und/oder ermittelbar auf das Volumen und/oder den Druck im zweiten Fluidraum (22) auswirkt, wobei die medizinische Fluidfödervorrichtung derart ausgebildet ist, dass aufgrund von während eines Zeitraums an der Pumpeinheit (4, 6) anliegenden Signalen und/oder einer mit der Pumpeinheit (4, 6) gekoppelten Sensorik das dem Fluidraum (14, 22) während dieses Zeitraums hinzugefügte oder entzogene Fluidvolumen ableitbar ist, **gekennzeichnet durch** einen zumindest abschnittsweise flexiblen Fluidaufnahmekörper (40), der in den zweiten Fluidraum (22) einsetzbar ist, um die zweite Trennwand (20b) zu bilden, die mit der beweglichen Trennwand (20a) in Anlage ist und der an eine Ventileinrichtung (34, 36) fluidangeschlossen ist, wobei der flexible Fluidaufnahmekörper (40) ein medizinischer Schlauch oder ein zumindest teilflexibler Beutel ist

2. Medizinische Fluidfördervorrichtung nach Anspruch 1, **gekennzeichnet durch**

- mindestens eine mit dem zweiten Fluidraum (22) verbundene Saugleitung (28) mit zugehöriger Ventileinrichtung (34) und

- mindestens eine mit dem zweiten Fluidraum (22) verbundene Druckleitung (30) mit zugehöriger Ventileinrichtung (36), wobei sich ein Volumen- und/oder Massenstrom aus der Saugleitung in die Druckleitung des zweiten Fluidsystems (S2) und /oder umgekehrt im ersten Fluidsystem (S1) erzeugen, ermitteln und steuern lässt.

3. Medizinische Fluidfördervorrichtung nach Anspruch 1, **dadurch gekennzeichnet, dass** es sich bei beiden Trennwänden (20a, 20b) um Membrane oder membranähnliche Trennwände handelt.

4. Medizinische Fluidfördervorrichtung nach einem der vorhergehenden Ansprüche, **dadurch gekennzeichnet, dass** bei geöffnetem stromaufwärtigen Ventil (34) und geschlossenem stromabwärtigen Ventil (36) eine Verkleinerung des Fluidvolumens im ersten Fluidraum (14) um $\Delta V$ im Wesentlichen zu einer Vergrößerung des Fluidvolumens im zweiten Fluidraum (22) um $\Delta V$ führt und dieses Fluidvolumen $\Delta V$ im Wesentlichen einem stromaufwärts an die Saugleitung (28) angeschlossenem Fluidsystem entnommen wird

5. Medizinische Fluidfördervorrichtung nach einem der vorhergehenden Ansprüche, **dadurch gekennzeichnet, dass** bei geschlossenem stromaufwärtigen Ventil (34) und geöffnetem stromabwärtigen Ventil (36) eine Vergößerung des Fluidvolumens im ersten Fluidraum (14) um $\Delta V$ im Wesentlichen zu einer Verkleinerung des Fluidvolumens im zweiten Fluidraum (22) um $\Delta V$ führt und dieses Fluidvolumen $\Delta V$ im Wesentlichen dem stromabwärts an die Druckleitung (30) angeschlossenem Fluidsystem hinzugefügt wird.

6. Medizinische Fluidfördervorrichtung nach einem der vorhergehenden Ansprüche, **dadurch gekennzeichnet, dass** sie mindestens einen Drucksensor (18) aufweist, der mit dem ersten Fluidraum (14) hydraulisch und/oder pneumatisch verbunden ist.

7. Medizinische Fluidfördervorrichung nach Anspruch 6, **dadurch gekennzeichnet, dass** sie mindestens einen Volumen- und/oder Massenstromsensor im ersten Fluidsystem (S16) aufweist, mittels dem/denen das dem Fluidraum (14) während eines Zeitraums hinzugefügte oder entzogene Fluidvolumen ermittelbar ist.

8. Medizinische Fluidfördervorrichtung nach einem der vorstehenden Ansprüche, **dadurch gekennzeichnet, dass** der erste Fluidraum (14) in einem ersten Behälter (12) ausgebildet ist und der zweite Fluidraum (22) in einem zweiten Behälter (24) ausgebildet ist, der mit dem ersten Behälter (12) mechanisch unter Zwischenlage der beweglichen Trennwände (20a/, 20b) verbunden ist.

9. Medizinische Fluidfördervorrichtung nach einem der vorstehenden Ansprüche, **dadurch gekennzeichnet, dass** das zweite System (S2) in Gänze oder zumindest die mit Fluid unmittelbar in Kontakt stehenden Bauteile des zweiten Systems (S2) als Einwegartikel konzipiert sind.

10. Medizinische Fluidfördervorrichtung nach Anspruch 1, **dadurch gekennzeichnet, dass** sie derart gestaltet ist, dass sich die Kopplung der beiden Trennwände (20a, 20b) bewerkstelligen, unterstützen, aufrechterhalten, verbessern, überprüfen und/oder aufheben lässt, indem der Druck in einem Trennwandzwischenraum, der zumindest auch durch Teile beider Trennwände begrenzt wird, überprüft und geregelt wird.

**Claims**

1. A medical fluid conveyor device, comprising

- a first fluid system (S1) consisting of a motor-driven pumping unit (4, 6) and a first fluid space (14), the pumping unit (4, 6) being capable of delivering fluid to and removing fluid from said first fluid space, and a first movable and/or deformable separating wall (20a), and
- a second fluid system (S2) which can be coupled to the first fluid system (S1) and consists of a second fluid space (22) and a second movable and/or deformable separating wall (20b) which can be mechanically or fluidically coupled to the first separating wall (20a) in such a manner that a change in volume and/or pressure in the first fluid space (14) has an essentially predictable and/or determinable impact on the volume and/or the pressure in the second fluid space (22),

wherein the medical fluid conveyor device is configured such that based on signals existing in a period of time on the part of the pumping unit (4, 6) and/or a sensor system coupled to the pumping unit (4, 6), the fluid volume added to the fluid space (14, 22) or withdrawn from the fluid space (14, 22) during this period of time can be derived, **characterized by** a fluid receiving body (40) which is flexible at least in portions and which can be inserted in the second fluid space (22) to form the second separating wall (20b) which is in contact with the movable separating wall (20a) and to which a valve means (34, 36) is fluidically connected, wherein

the flexible fluid receiving body (40) is a medical hose or a bag which is flexible at least in parts.

2. The medical fluid conveyor device according to claim 1, **characterized by**

   - at least one suction line (28) which is connected to the second fluid space (22) and comprises an associated valve means (34) and
   - at least one pressure line (30) which is connected to the second fluid space (22) and comprises an associated valve means (36),

   a volumetric flow and/or mass flow from the suction line into the pressure line of the second fluid system (S2) and/or vice versa, being able to be generated, determined and controlled in the first fluid system (S1).

3. The medical fluid conveyor device according to claim 1, **characterized in that** both separating walls (20a, 20b) are membranes or membrane-like separating walls.

4. The medical fluid conveyor device according to any of the preceding claims, **characterized in that** in the event of an opened upstream valve (34) and a closed downstream valve (36), a reduction of the fluid volume in the first fluid space (14) by $\Delta V$ essentially results in an enlargement of the fluid volume in the second fluid space (22) by $\Delta V$, said fluid volume $\Delta V$ being essentially taken from a fluid system connected to the suction line (28) at an upstream point.

5. The medical fluid conveyor device according to any of the preceding claims, **characterized in that** in the event of a closed upstream valve (34) and an opened downstream valve (36), an enlargement of the fluid volume in the first fluid space (14) by $\Delta V$ essentially results in a reduction of the fluid volume in the second fluid space (22) by $\Delta V$, said fluid volume $\Delta V$ being essentially added to a fluid system connected to the pressure line (30) at a downstream point.

6. The medical fluid conveyor device according to any of the preceding claims, **characterized in that** it comprises at least one pressure sensor (18) which is hydraulically and/or pneumatically connected to the first fluid space (14).

7. The medical fluid conveyor device according to claim 6, **characterized in that** it comprises at least one volumetric flow sensor and/or mass flow sensor in the first fluid system (S16), said sensor(s) allowing to determine the fluid volume which is added to or removed from the fluid space (14) during a period of time.

8. The medical fluid conveyor device according to any of the preceding claims, **characterized in that** the first fluid space (14) is formed in a first container (12) and the second fluid space (22) is formed in a second container (24) which is mechanically connected to the first container (12) by interposing the movable separating walls (20a, 20b).

9. The medical fluid conveyor device according to any of the preceding claims, **characterized in that** the second system (S2) in its entirety is designed as a disposable item or **in that** at least those components of the second system (S2) are designed as disposable items which are directly in contact with the fluid.

10. The medical fluid conveyor device according to claim 1, **characterized in that** it has such a design that the coupling of the two separating walls (20a, 20b) can be effected, supported, maintained, improved, checked and/or unloosened by checking and controlling the pressure in a separating wall interspace which is also delimited at least by partial areas of both separating walls.

**Revendications**

1. Dispositif médical de transport de fluide, comprenant

   - un premier circuit de fluide (S1), constitué d'une unité de pompe (4, 6) à entraînement motorisé et d'un premier compartiment de fluide (14), dans lequel le fluide peut être introduit et dont le fluide peut être prélevé par l'unité de pompe (4, 6), et une paroi de séparation (20a), mobile et/ou déformable, et

- un deuxième circuit de fluide (S2), pouvant être couplé au premier circuit de fluide (S1), constitué d'un deuxième compartiment de fluide (22) et d'une deuxième paroi de séparation mobile et/ou déformable (20b), qui par une liaison mécanique ou fluidique peut être couplée à la première paroi de séparation (20a), de telle sorte qu'une variation du volume et/ou de la pression dans le premier compartiment de fluide (14) agisse pour l'essentiel d'une manière prévisible et/ou déterminable sur le volume et/ou la pression dans le deuxième compartiment de fluide (22),

le dispositif médical de transport de fluide étant conçu de telle sorte que, sur la base de signaux appliqués pendant un certain laps de temps à l'unité de pompe (4, 6), et/ou d'un système de capteurs couplé à l'unité de pompe (4, 6), on puisse en déduire le volume de fluide introduit dans le compartiment de fluide (14, 22) pendant ce laps de temps, ou qui en a été prélevé,
**caractérisé par** au moins un corps récepteur de fluide (40), flexible au moins par secteurs, qui peut être inséré dans le deuxième compartiment de fluide (22), pour former la deuxième paroi de séparation (20b), qui s'appuie contre la paroi de séparation mobile (20a) et qui est raccordé par liaison fluidique à un dispositif de soupape (34, 36), le corps flexible récepteur de fluide (40) étant un tuyau souple médical ou une poche au moins partiellement flexible.

2. Dispositif médical de transport de fluide selon la revendication 1, **caractérisé par**

- au moins une conduite d'aspiration (28), reliée au deuxième compartiment de fluide (22), avec un dispositif de soupape correspondant (34), et
- au moins une conduite de refoulement (30), reliée au deuxième compartiment de fluide (22), avec le dispositif de soupape correspondant (36),

dans lequel un débit volumique et/ou massique, provenant de la conduite d'aspiration, pénétrant dans la conduite de refoulement du deuxième circuit de fluide (S2) et/ou inversement, peut être produit, déterminé et piloté dans le premier circuit de fluide (S1).

3. Dispositif médical de transport de fluide selon la revendication 1, **caractérisé en ce que**, pour ce qui concerne les deux parois de séparation (20a, 20b), il s'agit de membranes ou de parois de séparation analogues à des membranes.

4. Dispositif médical de transport de fluide selon l'une des revendications précédentes, **caractérisé en ce que**, quand la soupape amont (34) est ouverte et que la soupape aval (36) est fermée, une diminution de ΔV du volume de fluide dans le premier compartiment de fluide (14) conduit pour l'essentiel à une augmentation de ΔV du volume de fluide dans le deuxième compartiment de fluide (22), ce volume de fluide ΔV étant prélevé pour l'essentiel d'un circuit de fluide raccordé en amont à la conduite d'aspiration (28).

5. Dispositif médical de transport de fluide selon l'une des revendications précédentes, **caractérisé en ce que**, quand la soupape amont (34) est fermée et que la soupape aval (36) est ouverte, une augmentation de ΔV du volume de fluide dans le premier compartiment de fluide (14) conduit pour l'essentiel à une diminution de ΔV du volume de fluide dans le deuxième compartiment de fluide (22), et ce volume de fluide ΔV étant pour l'essentiel ajouté au circuit de fluide raccordé en aval de la conduite de refoulement (30).

6. Dispositif médical de transport de fluide selon l'une des revendications précédentes, **caractérisé en ce qu'**il comprend au moins un capteur de pression (18), qui est relié par une liaison hydraulique et/ou pneumatique au premier compartiment de fluide (14).

7. Dispositif médical de transport de fluide selon la revendication 6, **caractérisé en ce qu'**il comprend au moins un capteur de débit volumique et/ou de débit massique dans le premier circuit de fluide (S16), à l'aide duquel/desquels il est possible de déterminer le volume de fluide introduit dans le compartiment de fluide (14) pendant un certain laps de temps, ou prélevé de ce dernier.

8. Dispositif médical de transport de fluide selon l'une des revendications précédentes, **caractérisé en ce que** le premier compartiment de fluide (14) est configuré dans un premier récipient (12), et le deuxième compartiment de fluide (22) dans un deuxième récipient (24), qui est mécaniquement relié au premier récipient (12), avec interposition des parois de séparation mobiles (20a/20b).

9. Dispositif médical de transport de fluide selon l'une des revendications précédentes, **caractérisé en ce que** le deuxième circuit (S2) dans sa totalité, ou au moins les composants du deuxième circuit (S2) qui sont en contact direct avec le fluide, sont conçus comme dés articles à usage unique.

10. Dispositif médical de transport de fluide selon la revendication 1, **caractérisé en ce qu'**il est configuré de telle sorte que le couplage des deux parois de séparation (20a, 20b) puisse être réalisé, soutenu, maintenu, amélioré, contrôlé et/ou supprimé par un contrôle et une régulation de la pression dans un espace intermédiaire entre les parois de séparation, qui au moins aussi est délimité par des parties des deux parois de séparation.

# Fig.1

Abb.1

Abb.2

Abb.3

# Fig.2

Abb.1

Abb.2

# Fig.3

Abb.1

A-A

B-B

Abb.2

Abb.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120141306 A
- US 6250502 B1
- EP 0191071 B1
- US 5921951 A
- WO 2006000415 A1
- DE 19919572 A1